# EUROPEAN PATENT APPLICATION

(11) **EP 1 560 145 A2**
(43) Date of publication of application: **03.08.2005**
(21) Application number: 05002054.4
(22) Date of filing: 01.02.2005
(51) Int. Cl.: G06F 19/00

(54) **Biological data acquiring apparatus and storage device for biological data acquiring apparatus**

(30) Priority: 02.02.2004 JP 2004026113
(71) Applicant: TANITA CORPORATION, Tokyo (JP)
(72) Inventor: Miyashita, Yuichi c/o TANITA CORPORATION, Tokyo (JP)
(74) Representative: Grünecker, Kinkeldey, Stockmair & Schwanhäusser Anwaltssozietät

(57) **Abstract**

There is provided a biological data acquiring apparatus which can reduce a possibility that biological data stored in a biological data storing section is deleted against a user's intention. The biological data acquiring apparatus according to the present invention comprises a biological data acquiring section for acquiring biological data, a biological data storing section for storing acquired biological data sequentially and a control section for controlling these biological data acquiring section and biological data storing section, the apparatus further comprises a space information storing section for storing information about the storage space of the biological data storing section and a storage state informing section for informing information about the storage state of the biological data storing section, and the control section updates the information about the storage space each time it stores biological data in the biological data storing section and outputs the information about the storage state to the storage state informing section based on the updated information about the storage space.

## Description

### BACKGROUND OF THE INVENTION

(i) Field of the Invention This invention relates to a biological data acquiring apparatus for acquiring biological data of a user and a storage device for the biological data acquiring apparatus. Particularly, it relates to a biological data acquiring apparatus which has a biological data storing section for storing acquired biological data and a storage device for the biological data acquiring apparatus.
(ii) Description of the Related Art
   Various biological data acquiring apparatuses which acquire biological data of a user such as a body weight, a body fat percentage, pulses and the number of steps have been developed and practically used. These biological data acquiring apparatuses include those which directly measure a body weight which is biological data of a user like a scale and those which calculate desired biological data such as a body fat mass (percentage), a total body water (percentage), a muscle mass (percentage), a visceral fat area, a bone density and a basal metabolic rate from measured biological data of a user, i.e., a body weight and a bioelectrical impedance value, and biological data entered by the user, e.g., a body height, age and gender, like a body fat monitor (for example, refer to Patent Publications 1 and 2).

Further, as such biological data acquiring apparatuses, those which store acquired biological data in a biological data storing section to, for example, allow a user to check changes in biological data within a given period are also known (for example, refer to Patent Publication 3).

Further, in recent years, there are systems which make it possible to, for example, manage the long-term continuous change record of biological data, by sending biological data acquired by these biological data acquiring apparatuses to an external data processing device such as a personal computer and storing the biological data in the external device. As such systems, there are proposed systems which acquire biological data of a user such as the number of steps and a walking pace by use of a body motion sensor and a computing unit which are incorporated in a pedometer, send the biological data to a personal computer by means of a communication cable or an optical communication unit and perform calculations on the biological data and other biological data entered in the personal computer such as the body height, body weight, age and gender of the user to acquire biological data such as consumed calories and change records thereof (refer to Patent Publication 4).

### Patent Publication 1

Japanese Patent Publication No. 5-49050

### Patent Publication 2

Japanese Patent Laid-Open Publication No. 2001-70273

### Patent Publication 3

Japanese Patent Laid-Open Publication No. 2001-190514

### Patent Publication 4

Japanese Patent Laid-Open Publication No. 2000-41953

Of the above conventional biological data acquiring apparatuses, particularly, in biological data acquiring apparatuses of a type which stores acquired biological data in a biological data storing section, when biological data are stored in the biological data storing section to its full storage capacity, past biological data stored therein are automatically overwritten and deleted, generally from the oldest biological data, by newly acquired biological data, whereby the newly acquired biological data is stored.

Further, biological data acquiring apparatuses of a type which sends acquired biological data to an external data processing device and stores the acquired biological data in the device often store the acquired biological data in the biological data storing section thereof temporarily. In such a case as well, when biological data are stored in the biological data storing section to its full storage capacity, stored biological data are automatically overwritten and deleted by newly acquired biological data, whereby the newly acquired biological data is stored.

If the biological data acquiring apparatuses are constituted such that past biological data stored in the biological data storing section are automatically deleted as described above when biological data are stored in the biological data storing section to its full storage capacity, the past biological data may be deleted against a user's intention even if the user does not desire deletion of the biological data. In particular, in the case of a biological data acquiring apparatus which is so constituted as not to be allowed to store a large amount of biological data in the biological data storing section thereof on the premise that biological data are sent to an external data processing device at a relatively short time interval (e.g., once in a week), if a user acquires biological data without performing the above data transmission at a certain time interval unintentionally, past biological data may be deleted and the above long-term continuous change record may be adversely affected.

Therefore, an object of the present invention is to provide a biological data acquiring apparatus which can reduce a possibility that biological data stored in a biological data storing section is deleted against a user's intention and a storage device for such a biological data acquiring apparatus.

Further, a secondary object of the present invention is to provide the biological data acquiring apparatus and storage device which can delete stored biological data and store the most recent biological data when the user permits deletion of the stored biological data.

### SUMMARY OF THE INVENTION

A biological data acquiring apparatus according to the present invention comprises:
a biological data acquiring section for acquiring biological data,
a biological data storing section for storing acquired biological data sequentially, and
a control section for controlling these biological data acquiring section and biological data storing section, the apparatus further comprising:
   a space information storing section for storing information about the storage space of the biological data storing section, and
   a storage state informing section for informing information about the storage state of the biological data storing section, the control section updating the information about the storage space each time it stores biological data in the biological data storing section and outputting the information about the storage state to the storage state informing section based on the updated information about the storage space.

Further, the biological data acquiring apparatus according to the present invention is desirably such that when the information about the storage space has reached a predetermined state indicating that the biological data storing section has no available space left by the updating of the information about the storage space, the control section outputs information indicating that the biological data storing section has no available space left to the storage state informing section as the information about the storage state and does not perform updating of the information about the storage space.

Further, the biological data acquiring apparatus according to the present invention is desirably such that the apparatus further comprises an order information storing section containing information about the order of storage of biological data into the biological data storing section, and the control section updates the information about the order of storage each time it stores biological data in the biological data storing section and stores the biological data in the biological data storing section based on the updated information about the order of storage.

Further, the biological data acquiring apparatus according to the present invention is desirably such that the apparatus further comprises biological data sending means for sending biological data stored in the biological data storing section to an external data processing device, and the control section resets the information about the storage space to an initial state when the biological data stored in the biological data storing section is sent to the external data processing device by the biological data sending means.

Further, the biological data acquiring apparatus according to the present invention is desirably such that the biological data sending means is a storage device which is independent of and detachable from the apparatus's main unit incorporating at least the biological data acquiring section and incorporates at least the biological data storing section and the space information storing section.

Further, the biological data acquiring apparatus according to the present invention is desirably such that the storage device further incorporates the order information storing section.

Further, the biological data acquiring apparatus according to the present invention is desirably such that the apparatus further comprises a biological data displaying section for displaying biological data acquired by the biological data acquiring section, and the storage state informing section is integrated in the biological data displaying section.

Alternatively, a storage device for a biological data acquiring apparatus according to the present invention is independent of and detachable from the main unit of a biological data acquiring apparatus which incorporates a biological data acquiring section for acquiring biological data and incorporates a biological data storing section for storing biological data acquired by the biological data acquiring section and a space information storing section containing information about the storage space of the biological data storing section.

Further, the storage device for a biological data acquiring apparatus according to the present invention is desirably such that the device further comprises an order information storing section containing information about the order of storage of biological data into the biological data storing section.

According to the biological data acquiring apparatus of the present invention, since information about the storage state of the biological data storing section is informed based on information about the storage space of the biological data storing section which is updated each time biological data is stored, a user can recognize the storage state of biological data in the biological data storing section. Therefore, a possibility that deletion of stored biological data is performed against the user's intention can be reduced.

Further, if it is informed as the information about the storage state that the information about the storage space has been updated to a predetermined state indicating that the biological data storing section has no available space left and subsequent updating of the information about the storage space is cancelled, the user can be easily and always aware that currently stored biological data is overwritten and deleted if biological data is further acquired and stored. Thus, the possibility that deletion of stored biological data is performed against the user's intention can be reduced more securely.

Further, if biological data is stored in the biological data storing section based on information about the order of storage of biological data which is updated each time biological data is stored, biological data with the oldest storage order is overwritten and deleted by newly acquired biological data and the most recent biological data can be stored because the user permits deletion of already stored biological data when the user continues acquisition of biological data even if the biological data storing section has no available space left.

Further, if the information about the storage space is reset to an initial state when biological data stored in the biological data storing section are sent to an external data processing device by the biological data sending means, the information about the storage state which is informed after sending out the biological data can also be reset to an initial state. When this constitution is applied particularly to a constitution that it is informed as the information about the storage state that the biological data storing section has no available space left, the information about the storage state is not informed after sending out the biological data. Thus, unnecessary informing can be prevented, thereby attaining a rational biological data acquiring apparatus.

Further, when the biological data sending means is a storage device which is independent of and detachable from the main unit of the biological data acquiring apparatus and incorporates at least the biological data storing section and the space information storing section, preferably further incorporates the order information storing section, the biological data sending means can be inexpensive and used regardless of the positions of the main unit of the biological data acquiring apparatus and the data processing device, and a user-friendly biological data acquiring apparatus which can perform, e.g., informing of the above information about the storage space of the biological data storing section can be obtained.

Further, when the storage state informing section for informing the information about the storage state of the biological data storing section is integrated in the biological data displaying section for displaying biological data, it becomes possible that the information about the storage state of the biological data storing section is informed by use of the biological data displaying section which easily draws attention from the user. Thus, a possibility that the user overlooks the information about the storage state can be reduced.

Meanwhile, according to the storage device for the biological data acquiring apparatus of the present invention, since it is independent of and detachable from the main unit of the biological data acquiring apparatus and incorporates at least the biological data storing section and the space information storing section, the information about the storage state of the biological data storing section can be informed based on the information about the storage space which is stored in the space information storing section. Further, when the storage device further comprises the order information storing section, biological data can be stored in the biological data storing section based on the information about the order of storage which is stored in the order information storing section. Thus, there can be obtained a storage device for a biological data acquiring apparatus which can allow the user to recognize the storage state of biological data in the biological data storing section and allow newly acquired biological data to overwrite and delete biological data with the oldest storage order and be stored in the biological data storing section.

### BRIEF DESCRIPTION OF THE DRAWINGS

Fig. 1 is an external view of a biological data acquiring apparatus according to the present invention.
Fig. 2 is a schematic diagram illustrating the circuit configuration of the biological data acquiring apparatus according to the present invention.
Fig. 3 is a diagram illustrating the biological data storing section of the biological data acquiring apparatus according to the present invention.
Fig. 4 is a flowchart illustrating a control process executed by the main unit of the biological data acquiring apparatus according to the present invention.
Fig. 5 is a diagram wherein (a) to (e) show display examples of the liquid crystal display of the biological data acquiring apparatus according to the present invention.
Fig. 6 is a flowchart illustrating a control process executed by the storage device of the biological data acquiring apparatus according to the present invention.

### DETAILED DESCRIPTION OF THE PREFERRED EMBODIMENTS

A biological data acquiring apparatus according to the present invention comprises:
a biological data acquiring section for acquiring biological data,
a biological data storing section for storing acquired biological data sequentially, and
a control section for controlling these biological data acquiring section and biological data storing section,
the apparatus further comprising:
   a space information storing section for storing information about the storage space of the biological data storing section, and
   a storage state informing section for informing information about the storage state of the biological data storing section, the control section updating the information about the storage space each time it stores biological data in the biological data storing section and outputting the information about the storage state to the storage state informing section based on the updated information about the storage space.

Further, the biological data acquiring apparatus according to the present invention is desirably such that when the information about the storage space has reached a predetermined state indicating that the biological data storing section has no available space left by the updating of the information about the storage space, the control section outputs information indicating that the biological data storing section has no available space left to the storage state informing section as the information about the storage state and does not perform updating of the information about the storage space.

Further, the biological data acquiring apparatus according to the present invention is desirably such that the apparatus further comprises an order information storing section containing information about the order of storage of biological data into the biological data storing section, and the control section updates the information about the order of storage each time it stores biological data in the biological data storing section and stores the biological data in the biological data storing section based on the updated information about the order of storage.

Further, the biological data acquiring apparatus according to the present invention is desirably such that the apparatus further comprises biological data sending means for sending biological data stored in the biological data storing section to an external data processing device, and the control section resets the information about the storage space to an initial state when the biological data stored in the biological data storing section is sent to the external data processing device by the biological data sending means.

Further, the biological data acquiring apparatus according to the present invention is desirably such that the biological data sending means is a storage device which is independent of and detachable from the apparatus's main unit incorporating at least the biological data acquiring section and incorporates at least the biological data storing section and the space information storing section.

Further, the biological data acquiring apparatus according to the present invention is desirably such that the storage device further incorporates the order information storing section.

Further, the biological data acquiring apparatus according to the present invention is desirably such that the apparatus further comprises a biological data displaying section for displaying biological data acquired by the biological data acquiring section, and the storage state informing section is integrated in the biological data displaying section.

Alternatively, a storage device for a biological data acquiring apparatus according to the present invention is independent of and detachable from the main unit of a biological data acquiring apparatus which incorporates a biological data acquiring section for acquiring biological data and incorporates a biological data storing section for storing biological data acquired by the biological data acquiring section and a space information storing section containing information about the storage space of the biological data storing section.

Further, the storage device for a biological data acquiring apparatus according to the present invention is desirably such that the device further comprises an order information storing section containing information about the order of storage of biological data into the biological data storing section.

### Examples

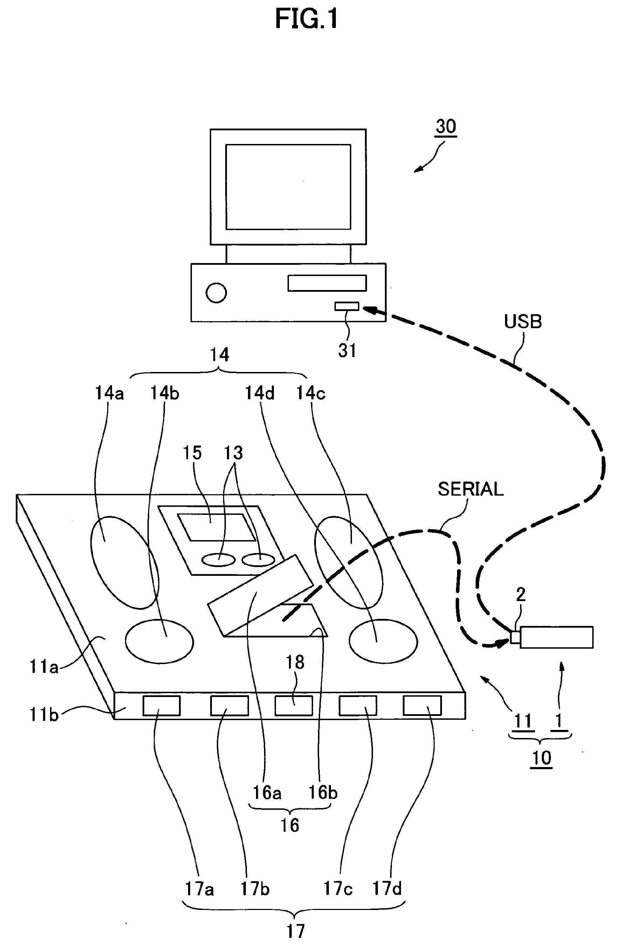

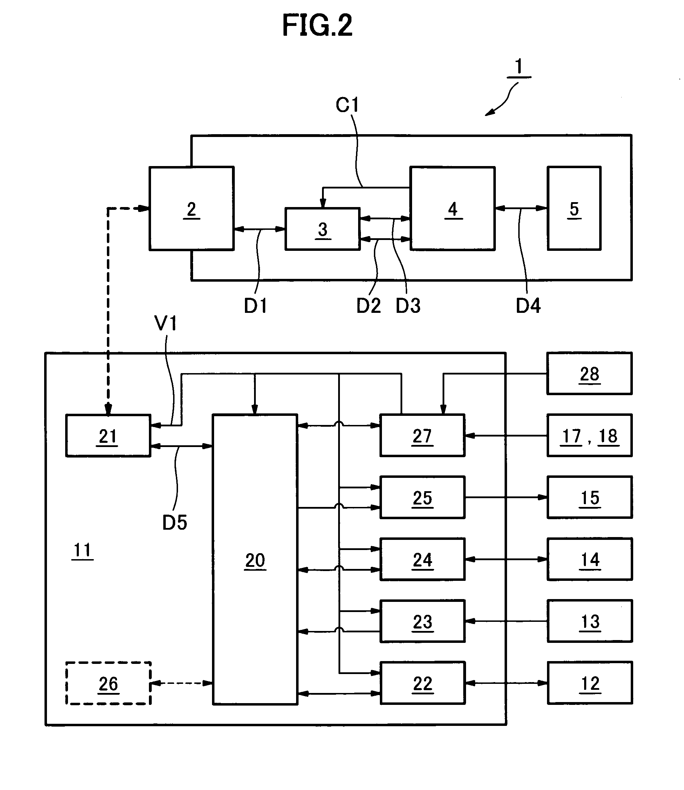

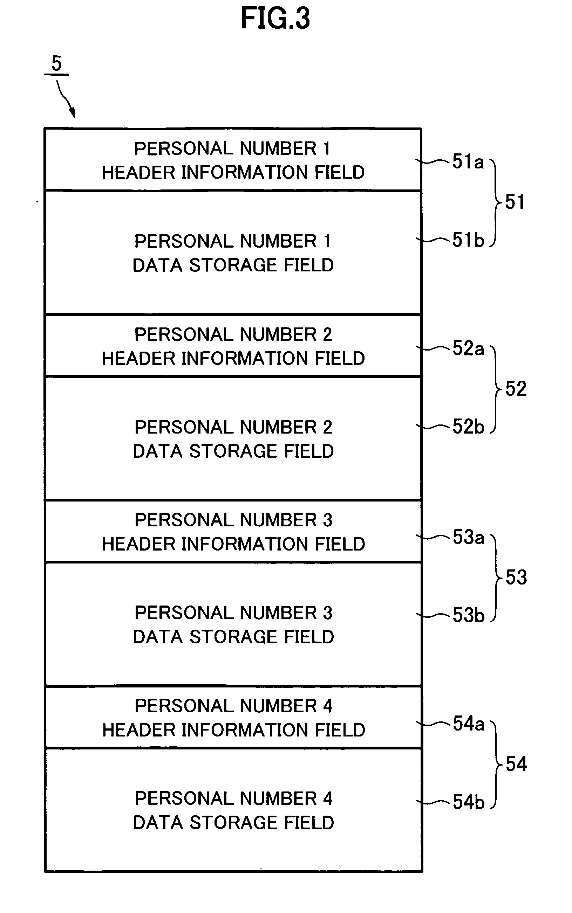

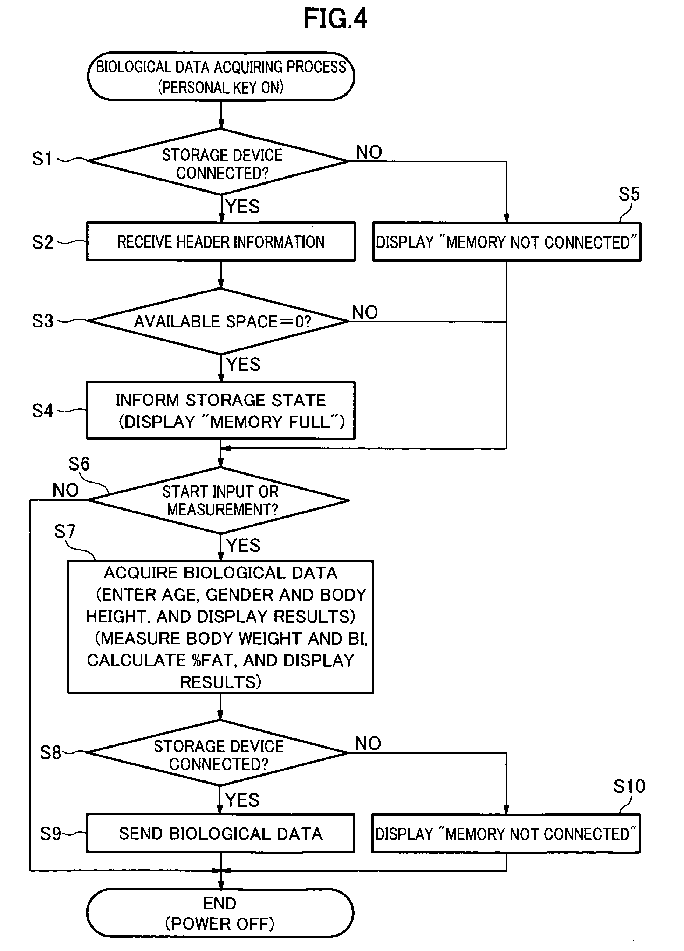

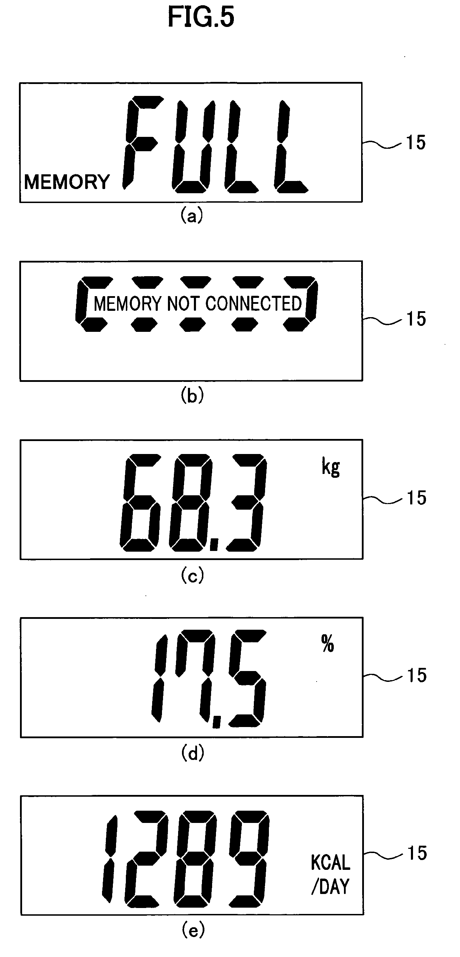

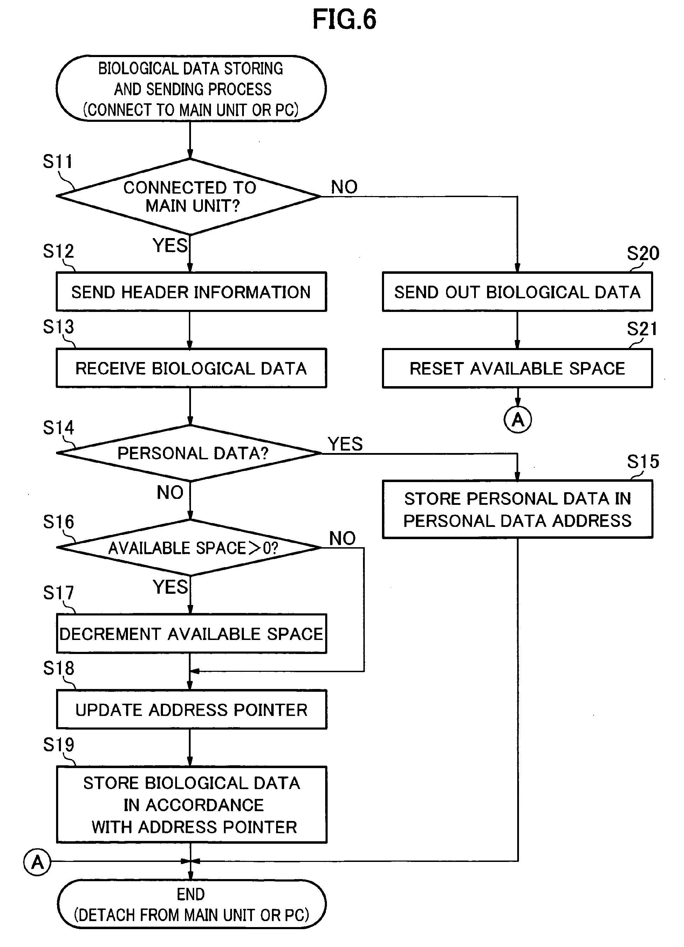

Hereinafter, an example of the present invention will be described with reference to the drawings. Fig. 1 is an external view of a biological data acquiring apparatus 10 as a suitable example of the present invention together with an external data processing device 30. Fig. 2 is a schematic diagram illustrating the circuit configuration of the biological data acquiring apparatus 10. Fig. 3 is a diagram illustrating the constitution of the biological data storing section of the biological data acquiring apparatus 10. Fig. 4 is a flowchart illustrating a control process executed by the main unit of the biological data acquiring apparatus 10. Fig. 5 is a diagram illustrating display examples of the liquid crystal display of the biological data acquiring apparatus 10. Fig. 6 is a flowchart illustrating a control process executed by the storage device of the biological data acquiring apparatus 10.

As shown in Figs. 1 and 2, the biological data acquiring apparatus 10 comprises a main unit 11 and a storage device 1 which is independent of and detachable from the main unit 11. The apparatus 10 can send biological data acquired in the main unit 11 of a user, such as age, gender, a body height, a body weight, a bioelectrical impedance, a body fat mass (percentage), a total body water (percentage), a muscle mass (percentage), a visceral fat area (level), a bone mass, a bone density, a basal metabolic rate and the like, to the external data processing device 30 via the storage device 1 as indicated by the dotted lines.

First of all, the main unit 11 is an improved version of a known so-called scale equipped with a body fat monitor which takes in biological data of a user such as age, gender and a body height, measures the body weight and bioelectrical impedance of the user and calculates the body fat mass (percentage), total body water (percentage), muscle mass (percentage), visceral fat area (level), bone mass, bone density, basal metabolic rate and the like, based on these entered and measured data. In the present example, the above biological data to be input will be generically referred to as "personal data", and the above biological data to be measured or calculated will be generically referred to as "measurement data" hereinafter.

This main unit 11 is basically constituted as a scale having a known load cell 12 (refer to Fig. 2) incorporated therein. On its top surface 11a, there are provided input switches 13 which are used by a user to input personal data, electrodes 14 which make contact with the bottoms of the feet of the user to measure a bioelectrical impedance, a liquid crystal display 15 which displays personal data and measurement data, and an installation section 16 in which the storage device 1 to be described later is installed. Further, on the side face 11b, there are provided personal key switches 17 which comprise four personal key switches 17a, 17b, 17c and 17d and a power-off switch 18. Each of the switches 17a to 17d can also serve as a power-on switch.

The electrodes 14 comprise electrodes 14a and 14b which make contact with the bottom of the left foot of the user and electrodes 14c and 14d which make contact with the bottom of the right foot of the user. Of these, the electrodes 14a and 14c which make contact with the toe sides of the feet are used to pass a weak current between the feet, and the electrodes 14b and 14d which make contact with the heel sides of the feet are used to measure a potential difference (voltage) between the feet. A bioelectrical impedance can be determined from these current value and voltage value.

Further, in the central portion between the electrodes 14a and 14b for the left foot and the electrodes 14c and 14d for the right foot of the top surface 11a of the main unit, the installation section 16 for the storage device 1 to be described later is formed. The installation section 16 is formed as a fitting cavity 16b of such a size that the storage device 1 to be described later can be fitted therein in a laid position. The cavity has an openable/closable cover 16a. On the internal surface of the fitting cavity 16b, a terminal 21 (refer to Fig. 2) which is used to connect the storage device 1 to the main unit is provided.

When the installation section 16 for the storage device 1 is formed on the top surface 11a of the main unit as described above, the storage device 1 can be attached and detached more easily than when the installation section 16 is formed on the side or rear surface of the main unit 11. Further, when the installation section 16 has the foregoing structure that the storage device 1 can be fitted therein in a laid position, it can be prevented that the storage device 1 sticks out from the top surface 11a at the time of its installation without increasing the thickness of the main unit 11 so much, thereby making it difficult to occur that a user mistakenly steps on the storage device 1 and breaks it. Further, with the openable/closable cover 16a, the possibility of occurrence of such a breakage is further reduced to a significant degree.

As a matter of course, the position and shape of the installation section 16 need not be limited to those in the present example and may be any appropriate position and shape according to the sizes and other features of the storage device 1 and main unit 11. For example, in the case of a biological data acquiring apparatus which has the input switches 13 and the liquid crystal display 15 as a unit which is detachable from or independent of the main unit 11, the installation section 16 may be formed in the unit.

Meanwhile, the storage device 1 is an improved version of a known so-called USB memory which has already been commercially available in various forms as an external memory for a personal computer having a terminal conforming to the USB (Universal Serial Bus) standard. The size and shape of the storage device 1 are nearly the same as those of a chewing gum package. It has a male USB terminal 2 at one end in its longitudinal direction and can be easily connected to the main unit 11 and the data processing device 30 which have a terminal corresponding to the USB terminal 2. Thereby, the storage device 1 exchanges data with these main unit 11 and data processing device 30 as host devices.

Further, the biological data acquiring apparatus 10 (main unit 11 and storage device 1) has a circuit configuration as shown in Fig. 2 therein.

First, in the main unit 11, a microcomputer 20 which comprises a CPU, a ROM and a RAM to accept input of personal data from a user and to measure or calculate measurement data is incorporated. To the microcomputer 20, the terminal 21 which corresponds to the USB terminal 2 of the storage device 1 is connected via a data bus D5. Further, a weighing circuit 22 which is connected to the above load cell 12, an input circuit 23 which is connected to the input switches 13, an impedance circuit 24 which is connected to the electrodes 14, a display output circuit 25 which is connected to the liquid crystal display 15, and a power supply circuit 27 which is connected to the personal key switches 17 and power-off switch 18 and a battery 28 are also connected to the microcomputer 20 via respective data buses or control signal buses. Further, these microcomputer 20, terminal 21 and circuits 22 to 25 are connected to the power supply circuit 27 via a power supply line VI.

Further, aside from the storage device 1, the main unit 11 may incorporate an EEPROM 26 for storing personal data and measurement data.

Further, the microcomputer 20 calculates a body fat percentage and other data, based on a body weight measured by the load cell 12 and the weighing circuit 22, personal data entered by the input switches 13 and the input circuit 23 and a bioelectrical impedance measured by the electrodes 14 and the impedance circuit 24. Further, the microcomputer 20 outputs entered personal data and measured or calculated measurement data (i.e., biological data) to the liquid crystal display 15 via the display output circuit 25 and displays the data on the liquid crystal display 15. Further, the microcomputer 20 sends these biological data to the storage device 1 via the terminal 21. In addition, the microcomputer 20 also performs various other operations, e.g. , timing and updating date and time data constantly by a built-in clock.

Meanwhile, the storage device 1 comprises the above USB terminal 2, an analog switch 3 which is used to select a data signal to be exchanged with the main unit 11 or the data processing device 30 as a host device, a microcomputer 4 which executes controls related to processing and storing of a data signal, and an EEPROM 5. As a matter of course, the EEPROM 5 may be replaced to any other rewritable storage medium capable of storing data such as a nonvolatile memory. For example, a flash memory may be used alternatively.

The USB terminal 2 is connected to the analog switch 3 via a data bus D1. The analog switch 3 is connected to the microcomputer 4 via data buses D2 and D3 and a control signal bus C1. The microcomputer 4 is connected to the EEPROM 5 via a data bus D4. The analog switch 3, the microcomputer 4 and the EEPROM 5 are connected to the USB terminal 2 via a power supply line (not shown). Therefore, this storage device 1 operates by receiving power supplied from the host device via the USB terminal 2, as in the case of many other devices which conform to the USB standard.

When the USB terminal 2 of the storage device 1 is connected to the terminal 21 of the main unit 11, biological data acquired in the main unit 11 are received by the microcomputer 4 via the USB terminal 2, data bus D1, analog switch 3 and data bus D2, and then stored in the EEPROM 5 via the data bus D4 from the microcomputer 4.

The CPU in the microcomputer 20 of the main unit 11 is not as high in processing power as a CPU incorporated in a personal computer or equivalent devices and consequently does not support data transmission conforming to the USB standard. Thus, transmission of data from the main unit 11 to the storage device 1 relies on serial transmission using a serial signal such as RS232C (as indicated by the dotted lines SERIAL in Fig. 1).

Meanwhile, the data processing device 30 shown in Fig. 1 is a so-called desktop personal computer which supports data transmission conforming to the USB standard and has a female USB terminal 31. Further, in the data processing device 30, history management software capable of, for example, graphically displaying the history of changes in biological data on the monitor and driver software for controlling the storage device 1 are installed.

When the history management software is activated in the data processing device 30 and the USB terminal 2 of the storage device 1 is connected to the USB terminal 31 of the data processing device 30, the microcomputer 4 of the storage device 1 reads out biological data stored in the EEPROM 5 via the data bus D4, and then sends the biological data to the data processing device 30 via the data bus D3, analog switch 3, data bus D1 and USB terminal 2 (as indicated by the dotted lines USB in Fig. 1). Then, in the data processing device 30, management of the history of the received biological data is performed in accordance with the history management software.

That is, in the biological data acquiring apparatus 10 of the present example, the load cell 12 and weighing circuit 22, the input switches 13 and input circuit 23, the electrodes 14 and impedance circuit 24 and the microcomputer 20 constitute a biological data acquiring section for acquiring biological data; the EEPROM 5 constitutes a biological data storing section for storing biological data; and the microcomputer 20 and the microcomputer 4 constitute a control section for controlling these biological data acquiring section and biological data storing section. Further, the liquid crystal display section 15 and the display output circuit 25 constitute a biological data displaying section for displaying biological data, and the storage device 1 constitutes biological data sending means for sending biological data to the external data processing device 30.

The EEPROM 5 as the biological data storing section may be not only incorporated into the storage device 1 which is independent of and detachable from the main unit 11 as in the present example but also incorporated directly into the main unit 11, as exemplified by the EEPROM 26 shown by the dotted lines in Fig. 2. Further, the biological data sending means may be constituted by a communication cable which connects the main unit 11 and the data processing device 30 to each other or wireless communication equipment using an infrared ray or radio waves. However, when the biological data sending means is constituted by the storage device 1 having the EEPROM 5 as the biological data storing section as in the present example, it can be placed at a site distant from the main unit 11 and the data processing device 30, as compared with when the biological data sending means is constituted by the communication cable or infrared communication equipment, and it can be an inexpensive device, as compared with when the biological data sending means is constituted by radio wave communication equipment.

Further, as program software to be installed in the data processing terminal 30, a variety of program software such as one which gives a user, for example, advice about health management based on received biological data and one which transmits these biological data to others (e.g., the user's doctor) via the Internet or other route can be conceived.

Next, a detailed description will be given to the EEPROM 5 as the biological data storing section. As shown in Fig. 3, in the EEPROM 5, four fields 51, 52, 53 and 54 which correspond to the above four personal key switches 17a, 17b, 17c and 17d, respectively, are formed. Further, the fields 51, 52, 53 and 54 comprise header information fields 51a, 52a, 53a and 54a and data storage fields 51b, 52b, 53b and 54b, respectively.

The header information fields 51a to 54a each store information comprising four items(hereinafter generically referred to as "header information"),i.e., the number of a corresponding personal key switch (hereinafter referred to as "personal number"), a value representing the total capacity of a corresponding data storage field (hereinafter abbreviated as "total capacity"), a value representing currently available space in the corresponding data storage field (hereinafter abbreviated as "available space"), and a data storage address pointer indicating where in the corresponding data storage field biological data is to be stored (hereinafter abbreviated as "address pointer"). That is, in the present example, the above available space corresponds to information about the storage space of the biological data storing section, and the above address pointer corresponds to information about the order of storage of biological data. Thus, the above header information field constitutes a space information storing section and an order information storing section.

Further, in each of the data storage fields 51b to 54b, a plurality of data storage addresses are formed for storing biological data acquired in the main unit 11 sequentially. In the present example, in each of the data storage fields 51b to 54b, one personal data storage address (hereinafter referred to as "personal data address") and ten measurement data storage addresses (hereinafter referred to as "measurement data addresses") are formed. Therefore, in this storage device 1, personal data for one user and measurement data for 10 measurements (for 10 days) can be stored per personal number.

The number of the fields 51 to 54 is not limited to and may be smaller or larger than four. Further, the number of the measurement data addresses formed in the data storage fields 51b to 54b is not limited to 10 and may be smaller or larger than ten.

Next, a control process executed by the main unit 11 of the biological data acquiring apparatus 10 will be described with reference to the flowchart of Fig. 4. Further, display examples of the liquid crystal display 15 will also be described with reference to Fig. 5.

When a user presses down any of the personal key switches 17a to 17d of the main unit 11, electrical power is supplied from the battery 28 to each section of the main unit 11 via the power supply circuit 27, and in the microcomputer 20, a personal number corresponding to the pressed personal key switch is specified and the control process in accordance with the flowchart of Fig. 4 is executed. In this case, it is assumed that the personal key switch 17a has been pressed down and a personal number 1 has been specified.

In STEP S1, the microcomputer 20 checks whether the storage device 1 is connected to the main unit 11. More specifically, the microcomputer 20 sends a predetermined connection confirmation command pulse to the storage device 1 via the data bus D5 and the terminal 21. Receiving this connection confirmation command pulse, the storage device 1 sends back a predetermined connection response command pulse to the main unit 11 immediately. Thus, if the microcomputer 20 receives the connection response command pulse within a very short predetermined time (for example, 100 milliseconds) after sending the connection confirmation command pulse, it determines that the storage device 1 is connected to the main unit 11 and proceeds to STEP S2.

In STEP S2, first, the microcomputer 20 sends a header information request command pulse for requesting header information corresponding to the personal number 1 to the storage device 1. Receiving this header information request command pulse, the storage device 1 specifies the header information field 51a storing the personal number 1, reads header information out of the field 51a, and sends back the information to the main unit 11. Then, the microcomputer 20 receives the header information corresponding to the personal number 1.

In STEP S3, the microcomputer 20 determines whether available space in the data storage field 51b is 0 (zero) from the header information it has received in STEP S2. In the present example, the initial value of the available space is set at 10, and each time biological data is stored in the data storage field 51b, it is basically decremented by 1 (refer to STEP S17 to be described later). Thus, if the available space is 0, it indicates that measurement data are already stored in all of the ten measurement data addresses formed in the data storage field 51b. That is, in the present example, a state when the available space is 0 corresponds to a predetermined state when the biological data storing section has no available space left. The microcomputer 20 proceeds to STEP S4 when the available space is 0.

In STEP S4, the microcomputer 20 displays characters "MEMORY FULL" indicating that the EEPROM 5 as the biological data storing section has no storage space left, on the liquid crystal display 15, as shown in Fig. 5(a).

That is, in the present example, the character display corresponds to the information about the storage state of the biological data storing section, and a storage state informing section for informing this information is integrated in the liquid crystal display 15. Since the liquid crystal display 15 also displays biological data as described above, it draws attention from a user easily. Therefore, by performing the character display with the liquid crystal display 15, the user can be securely informed of the storage state of the biological data storing section. In addition, by the character display, the user can easily recognize that the EEPROM 5 does not have any available space left and can expect that past measurement data already stored in the EEPROM 5 will be overwritten if measurements of body weight, body fat percentage and the like are still continued. Thus, when the user does not want the past measurement data to be deleted, the user can take appropriate steps, e.g., detaching the storage device 1 from the main unit 11 at this time and connecting the storage device 1 to the external data processing device 30 so as to send out the measurement data stored in the EEPROM 5 to the data processing device 30 (refer to STEPS S20 and S21 to be described later) or replacing the storage device 1 by another storage device 1 having some available space left.

Means for informing the information about the storage state of the biological data storing section is not limited to character display as shown in Fig. 5(a). A more simplified symbol may be displayed in place of characters. Further, the storage state informing section may not have to be integrated in the liquid crystal display 15 and may be provided, for example, as an LED which lights upon informing, in the vicinity of the input switches 13 or on the storage device 1 itself. In addition, the storage state informing section is not limited to those appealing to the visual sense of a user and may also be those appealing to the auditory sense of the user, e. g. , a buzzer which gives a beep sound. It may also be a proper combination of these character display, LED, buzzer and the like.

After STEP S4, the microcomputer 20 proceeds to STEP S6 to prepare for input or measurement of biological data by the user.

Meanwhile, if the microcomputer 20 does not receive the connection response command pulse in the above STEP S1, it determines that the storage device 1 is not connected to the main unit 11 and proceeds to STEP S5. In STEP S5, the microcomputer 20 displays characters "MEMORY NOT CONNECTED" indicating the storage device 1 is not connected to the main unit 11, on the liquid crystal display 15, as shown in Fig. 5 (b) . This character display may also be replaced by more simplified symbol display, an LED, a buzzer or the like. Then, the microcomputer 20 proceeds to STEP S6 to prepare for input or measurement of biological data by the user.

Further, if the available space is not 0 (that is, the EEPROM 5 still has some available space left) in the above STEP S3, the microcomputer 20 skips STEP S4 and proceeds to STEP S6 to prepare for input or measurement of biological data by the user.

In STEP S6, the microcomputer 20 stands by for the start of input of personal data or the start of measurement of measurement data by the user for a predetermined standby time (e.g., 2 minutes). If no input is made from either the input switches 13 or the load cell 12 and the electrodes 14 within the standby time, the microcomputer 20 skips all subsequent steps to end this control process and shuts off power. On the other had, if inputs are made from either the input switches 13 or the load cell 12 and the electrodes 14, the microcomputer 20 proceeds to STEP S7.

In STEP S7, the microcomputer 20 not only acquires biological data but also displays the acquired biological data on the liquid crystal display 15. More specifically, when inputs are made from the input switches 13 in STEP S6, the microcomputer 20 acquires personal data in accordance with the inputs and displays the data in turn. Meanwhile, when inputs are made from the load cell 12 and the electrodes 14 in STEP S6, the microcomputer 20 measures a body weight and a bioelectrical impedance based on these inputs. Then, the microcomputer 20 reads out personal data stored in the data storage field 51a of the EEPROM 5 from the connected storage device 1 and calculates a body fat percentage and other data based on these body weight, bioelectrical impedance and personal data. The thus acquired measurement data are displayed in turn on the liquid crystal display 15, as exemplified in Figs. 5(c) to 5(e). Fig. 5(c) is a display example of a body weight, Fig. 5(d) is a display example of a body fat percentage, and Fig. 5(e) is a display example of a basal metabolic rate. Displays of these measurement data are repeated for a predetermined number of times in a given time. In this case, the body fat percentage and other data are not calculated when the storage device 1 is not connected to the main unit 11 or when personal data are not yet stored in the EEPROM 5.

In STEP S8, the microcomputer 20 rechecks connection of the storage device 1 in the same manner as in the above STEP S1. If it has been confirmed that the storage device 1 is connected to the main unit 1, the microcomputer 20 proceeds to STEP S9. If it has not been confirmed, the microcomputer 20 proceeds to STEP S10.

In STEP S9, the microcomputer 20 sends the biological data acquired in the above STEP S7 to the storage device 1 via the data bus D5 and the terminal 21. Then, after passage of a predetermined standby time (e.g., 2 minutes), the microcomputer 20 ends this control process and shuts off power.

In STEP S10, the microcomputer 20 displays "MEMORY NOT CONENCTED" on the liquid crystal display 15 as in the above STEP S5. Then, after passage of a predetermined standby time (e.g. , 2 minutes), the microcomputer 20 ends this control process and shuts off power.

The biological data acquired in the above STEP S7 are displayed continuously on the liquid crystal display 15 until the microcomputer 20 shuts off power after the above STEP S9 or STEP S10.

Next, a control process executed by the storage device 1 of the biological data acquiring apparatus 10 will be described with reference to the flowchart of Fig. 6. The microcomputer 4 in the storage device 1 is supplied with power required for its operation from the main unit 11 or the data processing device 30 as a host device.

In STEP S11, the microcomputer 4 checks whether the storage device 1 is currently connected to the main unit 11. More specifically, when the microcomputer 4 receives the above connection confirmation command pulse (refer to the above STEP S1) from the host device, it determines that the storage device 1 is connected to the main unit 11 and sends back the above connection response command pulse and proceeds to STEP S12. Meanwhile, when the microcomputer 4 receives a so-called bus reset signal (identification signal transmitted from a host device in data transmission conforming to the USB standard) from the host device, it determines that the storage device 1 is connected to the data processing device 30 and proceeds to STEP S20.

The storage device 1 in the present example has the analog switch 3 for data bus switching between the USB terminal 2 and the microcomputer 4. The microcomputer 4 controls the analog switch 3 via the control signal bus C1, thereby making it possible to use the data bus D2 for data transmission using a serial signal between the storage device 1 and the main unit 11 or to use the data bus D3 for data transmission conforming to the USB standard between the storage device 1 and the data processing device 30.

In STEP S12, the microcomputer 4 receives the header information request command pulse (refer to the above STEP S2) from the main unit 11, specifies the header information field 51a storing the corresponding personal number (in this case, the personal number 1) out of the EEPROM 5, reads out header information stored therein and sends back the information to the main unit 11.

In STEP S13, the microcomputer 4 receives the biological data sent from the main unit 11 (refer to the above STEP S9).

In STEP S14, the microcomputer 4 determines whether the biological data received in STEP S13 is personal data. If it is personal data, the microcomputer 4 proceeds to STEP S15.

In STEP S15, the microcomputer 4 stores the personal data received in STEP S14 in the personal data address formed in the data storage field 51b. If personal data is already stored therein, it is overwritten by the received personal data.

Meanwhile, if the biological data received in STEP S14 is not personal data but measurement data, the microcomputer 4 proceeds from STEP S14 to STEP S16.

In STEP S16, the microcomputer 4 determines whether available space in the data storage field 51b is 0 (zero) from the header information read in STEP S12. As described above, the initial value of the available space is set at 10, and each time biological data is stored in the data storage field, it is basically decremented by 1 (refer to STEP S17 to be described later). The microcomputer 4 proceeds to STEP S17 when the available space is not 0, while it skips STEP S17 and proceeds to STEP S18 when the available space is 0.

In STEP S17, the microcomputer 4 decrements the current available space by 1 and proceeds to STEP S18. When the available space becomes 0 as a result of the decrementation, the main unit 11 will go through the following operations the next time the storage device 1 is connected to the main unit 11. That is, the main unit 11 receives header information containing the updated available space in the above STEP S2, determines that the available space of the data storage field is 0 in the above STEP S3, and displays characters "MEMORY FULL" on the liquid crystal display 15 in the above STEP S4. Further, at this time, since this STEP S17 is skipped in the storage device 1, updating of the available space is not performed, thereby leaving the available space at 0. This available space is reset to the initial value when stored biological data are sent out to the data processing device 30 (refer to STEP S21 to be described later). Accordingly, the characters "MEMORY FULL" are displayed in the main unit 11 all the time until the user sends out the biological data.

In STEP S18, the microcomputer 4 updates an address pointer pointing out where in the data storage field 51b measurement data is to be stored. Since ten measurement data addresses are formed in each data storage field as described above, numerical values of 1 to 10 are given as the address pointers, for example. The address pointer is updated by repeating a sequence of numerical values 1, 2, 3, ... 9, 10 sequentially. That is, if the current address pointer is 10, this value is updated to 1.

For instance, when it is desired that measurement data for one day be stored in one measurement data address and biological data for a total of 10 days be stored in the storage device 1, it is to be programmed that date and time data counted/updated in the main unit 11 is included into measurement data, the date and time data in the measurement data to be stored this time is compared with date and time data in measurement data stored last time, and the address pointer is not updated when they match each other (that is, when they are measurement data for the same day). Thus, measurement data for the same day is stored in the same measurement data address.

In STEP S19, the microcomputer 4 stores the measurement data in a measurement data address specified by the address pointer. If measurement data is already stored therein, it is overwritten. As a result, when the user has continued measurement in the above STEP S6 despite displaying the characters "MEMORY FULL" on the liquid crystal display 15 in the above STEP S4, this implies that the user permits deletion of stored data, so that stored measurement data are overwritten and deleted in turn from the oldest measurement data.

In the present example, the process of storing the measurement data in the above STEP S19 is performed after the process of updating the available space in the above STEPS S16 and S17 and the process of updating the address pointer in the above STEP S18. However, the storing process may be carried out before these updating processes.

Meanwhile, if the storage device 1 is connected to the data processing device 30 in the above STEP S11, the microcomputer 4 proceeds to STEP S20 as described above.

In STEP S20, the microcomputer 4 sends biological data to the data processing device 30. More specifically, when the microcomputer 4 receives a biological data requesting signal sent from the data processing device 30 in accordance with the above history management software or the like, the microcomputer 4 sends personal data and measurement data stored in the data storage field to the data processing device 30 by means of data transmission conforming to the USB standard. Thereafter, the microcomputer 4 proceeds to STEP S21.

In STEP S21, the microcomputer 4 resets the available space in the data storage field which has sent out the biological data in STEP S20 to the initial value, i.e., 10. As a result, next time the storage device 1 is connected to the main unit 11, it is determined in the above STEP S3 that the available space is not 0, and "MEMORY FULL" will not be displayed.

Although a suitable example of the present invention has been described above, it is needless to say that various modifications as mentioned as appropriate in the description of the present example can be made to the present invention. Further, a biological data acquiring apparatus to which the present invention can be applied is not limited to a body-fat-monitor-incorporated scale of a type which measures a bioelectrical impedance between both feet as in the above example and may be a type which measures a bioelectrical impedance between both hands and/or between a hand and a foot or may also be a biological data acquiring apparatus other than the body-fat-monitor-incorporated scale, e.g., a pedometer, a blood pressure meter or a thermometer.

## Claims

1. A biological data acquiring apparatus comprising:
a biological data acquiring section for acquiring biological data,
a biological data storing section for storing acquired biological data sequentially, and
a control section for controlling these biological data acquiring section and biological data storing section, the apparatus further comprising:
a space information storing section for storing information about the storage space of the biological data storing section, and
a storage state informing section for informing information about the storage state of the biological data storing section, the control section updating the information about the storage space each time it stores biological data in the biological data storing section and outputting the information about the storage state to the storage state informing section based on the updated information about the storage space.

2. The apparatus of claim 1, wherein when the information about the storage space has reached a predetermined state indicating that the biological data storing section has no available space left by the updating of the information about the storage space, the control section outputs information indicating that the biological data storing section has no available space left to the storage state informing section as the information about the storage state and does not perform updating of the information about the storage space.

3. The apparatus of claim 1 or 2, further comprising an order information storing section containing information about the order of storage of biological data into the biological data storing section,
the control section updating the information about the order of storage each time it stores biological data in the biological data storing section and storing the biological data in the biological data storing section based on the updated information about the order of storage.

4. The apparatus of claims 1 to 3, further comprising biological data sending means for sending biological data stored in the biological data storing section to an external data processing device, the control section resetting the information about the storage space to an initial state when the biological data stored in the biological data storing section is sent to the external data processing device by the biological data sending means.

5. The apparatus of claim 4, wherein the biological data sending means is a storage device which is independent of and detachable from the apparatus's main unit incorporating at least the biological data acquiring section and incorporates at least the biological data storing section and the space information storing section.

6. The apparatus of claim 5, wherein the storage device further incorporates the order information storing section.

7. The apparatus of any one of claims 1 to 6, further comprising a biological data displaying section for displaying biological data acquired by the biological data acquiring section,
the storage state informing section being integrated in the biological data displaying section.

8. A storage device for a biological data acquiring apparatus, the storage device being independent of and detachable from the main unit of a biological data acquiring apparatus which incorporates a biological data acquiring section for acquiring biological data,
the storage device incorporating a biological data storing section for storing biological data acquired by the biological data acquiring section and a space information storing section containing information about the storage space of the biological data storing section.

9. The device of claim 8, further comprising an order information storing section containing information about the order of storage of biological data into the biological data storing section.
